# EUROPÄISCHE PATENTSCHRIFT

(11) **EP 4 333 767 B1**
(45) Veröffentlichungstag und Bekanntmachung des Hinweises auf die Patenterteilung: **30.09.2026**
(21) Anmeldenummer: 22728067.4
(22) Anmeldetag: 04.05.2022
(51) Int. Cl.: A61F 2/16, G02B 5/20, B29D 11/00, B29D 11/02

(54) **KÜNSTLICHE AUGENLINSE UND VERFAHREN ZU DEREN HERSTELLUNG**
ARTIFICIAL EYE LENS AND METHOD FOR PRODUCTION THEREOF
CRISTALLIN OCULAIRE ARTIFICIEL ET SON PROCÉDÉ DE PRODUCTION

(30) Priorität: 07.05.2021 DE 102021204662
(43) Veröffentlichungstag der Anmeldung: 13.03.2024
(73) Patentinhaber: Carl Zeiss Meditec AG, 07745 Jena (DE)
(72) Erfinder: SCHREIBER, Benjamin, 10589 Berlin (DE)
(74) Vertreter: Nieten, Christoph
(86) Internationale Anmeldenummer: PCT/EP2022/062003
(87) Internationale Veröffentlichungsnummer: WO 2022/233947

(56) Entgegenhaltungen:
- WO-A1-2008/036674
- WO-A1-98/56315
- DE-A1- 102010 017 240
- US-A1- 2008 269 890

## Beschreibung

Die vorliegende Erfindung betrifft eine künstliche Augenlinse für eine Implantation in einem Auge, umfassend ein Optikelement mit einer Vorderseite, einer Rückseite, einer optischen Zone und einer optischen Hauptachse, wobei die optische Zone zum Führen von Licht auf eine Netzhaut des Auges ausgebildet ist, und wobei das Optikelement ein erstes Linsenmaterial mit einem ersten Brechungsindex aufweist. Die Erfindung betrifft weiterhin ein Verfahren zur Herstellung einer solchen Augenlinse.

Zur Behandlung eines Kataraktes hat sich das Einsetzen von künstlichen Augenlinsen oder Intraokularlinsen (IOL) etabliert. Dabei wird die durch den Katarakt getrübte natürliche Augenlinse entfernt und durch eine Intraokularlinse ersetzt. Aber auch aus anderen Gründen kann das Einsetzen einer künstlichen Augenlinse notwendig sein. Typischerweise wird die Intraokularlinse im Kapselsack des Auges platziert. Dazu wird die vordere Kapselsackmembran geöffnet, die natürliche Augenlinse zerkleinert und entfernt und in den verbleibenden Kapselsack die künstliche Augenlinse eingesetzt. Dabei wird die Intraokularlinse über einen sogenannten Zugangsschnitt eingeführt, der eine Öffnung durch die Kornea bis in den Kapselsack bereitstellt. Die Breite des Zugangsschnittes hat Auswirkungen auf die nachfolgende Wundheilung sowie auf mögliche Komplikationen während der Implantation. Daher ist es wichtig, die Breite des Zugangsschnittes so gering wie möglich zu halten. Mittlerweile werden in einer sogenannten "Micro Incision Cataract Surgery" (MICS) Breiten für Zugangsschnitte von weniger als 2 mm benötigt. Durch die Verwendung von Linsenmaterialien mit hohem Brechungsindex können Intraokularlinsen mit geringer Linsenkrümmungen realisiert werden, die somit dünner und kleiner sind als Linsen gleicher Brechkraft mit geringerem Brechungsindex. Auf diese Weise werden die genannten schmalen Zugangsschnitte ermöglicht.

Nach einer Implantation einer künstlichen Augenlinse kann ein sogenannter Nachstar (oft "secondary cataract" oder "posterior capsule opaticty" (PCO) genannt) auftreten. Dabei handelt es sich typischerweise um Linsenepithelzellen, die sich vermehren und als dünne Lage von Zellen auf der der Retina zugewandten (posterioren) Seite der Intraokularlinse bis in die optische Zone hineinwachsen. Dabei ist unter der optischen Zone derjenige Bereich der künstlichen Augenlins zu verstehen, der für eine abbildenden Lichtführung auf die Retina des Auges ausgebildet ist. Die Zellen im Bereich der optischen Zone führen zu einer Eintrübung und somit zu einer Verschlechterung der Sicht.

Es ist bekannt, dass das Risiko für das Auftreten eines Nachstars verringert ist, wenn die Intraokularlinse eine Geometrie mit scharfen Kanten außerhalb der optischen Zone aufweist. Diese Kanten behindern ein Zellwachstum über die Kanten hinweg, so dass die Linsenepithelzellen nicht in den für eine abbildende Lichtführung ausgebildeten Bereich der künstlichen Augenlinse hineinwachsen können. Nachteil solcher Intraokularlinsen jedoch ist, dass sie ungewollte Lichtreflexe und Dysphotopsien verursachen. Ursache dafür ist, dass Licht, das beispielsweise unter großen Winkeln ins Auge fällt, an einer Kante der Intraokularlinse gestreut werden kann und somit an einen Ort auf der Retina gelenkt wird, der nicht der Abbildung in einem Auge ohne künstliche Augenlinse - d.h. mit natürlicher Augenlinse - entspricht. Diese Effekte treten vermehrt bei Intraokularlinsen mit hohem Brechungsindex auf und führen zu einer Verschlechterung der Sehleistung und somit zu Verringerung der Akzeptanz des Patienten für die künstliche Augenlinse.

In der Schrift US 2011/0060409 A1 wird eine Intraokularlinse vorgeschlagen, die an ihrem äußeren Rand konkave Einbuchtungen aufweist. Aufgrund von scharfen Kanten zur Vermeidung von Nachstar treten aber auch hier ungewollte Lichtreflexe auf.

In DE 10 2010 017240 A1 wird eine Intraokularlinse vorgeschlagen, die eine Schutzvorrichtung aufweist, um störenden Lichteinfach zu vermindern.

Aufgabe der vorliegenden Erfindung ist es, eine künstliche Augenlinse zu beschreiben, die es erlaubt, sowohl das Risiko für einen Nachstar zu verringern als auch das Auftreten von ungewollten Lichtreflexen und Dysphotopsien zu vermeiden.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Ein erster Aspekt der Erfindung betrifft eine künstliche Augenlinse für eine Implantation in einem Auge. Die künstliche Augenlinse umfasst ein Optikelement, das ein erstes Linsenmaterial mit einem ersten Brechungsindex aufweist. Bei dem Linsenmaterial kann es sich beispielsweise um Acryl, Silicon, oder Hydrogel handeln oder diese aufweisen. Das Linsenmaterial kann auch aus Polymethylmethacrylat (PMMA) bestehen (oder aufweisen) oder andere Materialien aufweisen, die in der Medizintechnik üblich sind, eine hohe Transmission aufweisen und biokompatibel sind. Der Brechungsindex des ersten Linsenmaterials ist typischerweise größer als der von Wasser oder Kammerwasser (etwa 1.33); typischerweise ist er größer als 1.4.

Das Optikelement weist weiterhin eine Vorderseite, eine Rückseite und eine optische Hauptachse auf. Dabei ist die Vorderseite des Optikelement im implantierten Zustand der Kornea des Auges zugewandt und die Rückseite des Optikelement ist der Netzhaut (Retina) zugewandt. Vorderseite und Rückseite sind als optische Flächen (auch optisch wirksame Flächen genannt) ausgeformt. Die Vorderseite und/oder die Rückseite können sphärisch oder asphärisch ausgeformt sein. Sie können weiterhin die Form einer Freiformfläche annehmen, d.h. sie können beispielsweise über ein Polynom oder stückweise über Polynome beschrieben sein. Die Vorderseite und/oder die Rückseite können zusätzlich diffraktive optische Strukturen aufweisen, um beispielsweise mehr als einen Brechkraft bereitzustellen. Die Vorderseite und die Rückseite der künstlichen Augenlinse sind für die optischen Abbildungseigenschaften verantwortlich. Licht kann auf der Vorderseite in die Augenlinse eindringen und diese an der Rückseite wieder verlassen. Die optische Hauptachse steht senkrecht auf einer gedachten Ebene, die sich zwischen der Vorderseite und der Rückseite der Augenlinse befindet. Die optische Hauptachse durchstößt das Optikelement typischerweise in den Scheitelpunkten der Vorderseite und der Rückseite.

Weiterhin umfasst das Optikelement eine optische Zone, wobei die optische Zone zum Führen von Licht auf die Netzhaut des Auges ausgebildet ist. Bei der optischen Zone handelt es sich um ein Teilvolumen des Optikelements. Ein Teil der Vorderseite und ein Teil der Rückseite begrenzen die optische Zone. Im implantierten Zustand der künstlichen Augenlinse kann Licht durch die Vorderseite in die optische Zone eindringen und diese auf der Rückseite des Optikelements wieder in Richtung Netzhaut verlassen. Dabei wird das Licht abbildend geführt. Derjenige Teil des Optikelements, der nicht zur optischen Zone gehört, dient im implantierten Zustand der künstlichen Augenlinse nicht einer abbildenden Lichtführung.

Die künstliche Augenlinse umfasst weiterhin ein Berandungselement, das mit dem Optikelement außerhalb der optischen Zone eine feste Verbindung aufweist. Das Berandungselement beeinträchtigt somit die abbildende Lichtführung der künstlichen Augenlinse nicht. Dabei weist das Berandungselement eine scharfe Kante in einer Schnittebene auf, die die optische Hauptachse umfasst. Unter einer Kante ist eine Unstetigkeit in der Krümmung der Oberfläche in der Schnittebene zu verstehen. Eine scharfe Kante liegt vor, wenn der in der Schnittebene innerhalb des Berandungselements gemessene Winkel an der Kante kleiner oder gleich 100° beträgt, bevorzugt kleiner oder gleich 90° (rechter Winkel). Da die Fertigung einer Kante Toleranzen unterliegt, die beispielsweise auf verwendete Werkzeuge zurückzuführen ist, kann der Winkel zwischen den Tangenten an Punkten des Berandungselements in 50 µm Abstand von einer idealen Kante (ohne Fertigungstoleranzen) bestimmt werden. Die scharfe Kante des Berandungselements befindet sich in der Schnittebene außerhalb der Verbindung des Berandungselements mit dem Optikelement. Die scharfe Kante der erfindungsgemäßen Augenlinse behindert ein Zellwachstum über diese scharfe Kante hinaus in Richtung der optischen Zone und verringert somit das Risiko eines Nachstars. Dazu befindet sich die scharfe Kante vorzugsweise auf der der Retina zugewandten (posterioren) Seite der künstlichen Augenlinse.

Erfindungsgemäß weist das Optikelement im Bereich der festen Verbindung in der Schnittebene keine scharfe Kante auf. Betrachtet man in der Schnittebene also den Bereich des Optikelement, an dem diese die feste Verbindung mit dem Berandungselement aufweist, so gibt es dort keine Kante, die einen Winkel (gemessen innerhalb des Optikelements) von 100° oder weniger aufweist, bzw. von 90° oder weniger. Bevorzugt weisen alle Kanten in diesem Bereich Winkel auf, die größer als 110° sind, besonders bevorzugt größer als 130°. Besonders bevorzugt weist das Optikelement in diesem Bereich keine Kante auf; es treten keine Unstetigkeiten in der Krümmung auf.

Weiterhin weist das Berandungselement der erfindungsgemäßen künstlichen Augenlinse ein zweites Linsenmaterial mit einem zweiten Brechungsindex auf. Dabei ist der zweite Brechungsindex vom ersten Brechungsindex verschieden. Betrachtet werden hier (und auch nachfolgend) die Brechungsindizes für sichtbares Licht - typischerweise für Wellenlängen von 400 nm bis 750 nm. Vorzugsweise unterscheiden sich die Brechungsindizes um mindestens 0.01, bevorzugt mindestens 0.05, besonders bevorzugt mindestens 0.1. Bei dem ersten und zweiten Linsenmaterial kann es sich um dieselbe Substanz handeln (z.B. beides Acryl); lediglich im Brechungsindex unterschieden sich die beiden Linsenmaterialien.

Durch die unterschiedlichen Brechungsindizes des ersten Linsenmaterials des Optikelements und des zweiten Linsenmaterials des Berandungselements ergibt sich beim Grenzübergang zwischen den beiden Materialien an deren fester Verbindung, dass kein Licht an der Grenzfläche gestreut oder ungewollt reflektiert werden kann, da das Optikelement im Bereich der festen Verbindung keine Scharfe Kante aufweist. Auf diese Weise können erfindungsgemäß sowohl ungewollte Lichtreflexe und Dysphotopsien vermieden als auch gleichzeitig aufgrund der scharfen Kante des Berandungselements außerhalb der festen Verbindung ein Zellwachstum behindert werden.

Die künstliche Augenlinse kann auch eine Haptik aufweisen. Die Haptik kann Teil des Berandungselements oder des Optikelements (außerhalb der optischen Zone) sein. Sie kann auch aus Teilen des Optikelements und des Berandungselements gebildet sein. Die Haptik ist dazu ausgebildet, im Auge fixiert zu werden, vorzugsweise im Kapselsack oder am Sulcus Ciliaris. Nach einer Implantation der künstlichen Augenlinse ins Auge und der Wundheilung wächst die Haptik ins Gewebe des Auges ein. Die Haptik kann beispielsweise als Plattenhaptik oder C-Loop Haptik ausgeformt sein.

Gemäß einer erfindungsgemäßen Ausgestaltung der künstlichen Augenlinse ist der erste Brechungsindex größer als der zweite Brechungsindex. Bevorzugt ist der der erste Brechungsindex um mindestens 0.01 größer als der zweite Brechungsindex, besonders bevorzugt mindestens 0.05, ganz besonders bevorzugt mindestens 0.1.

Bei künstlichen Augenlinsen nach dem Stand der Technik kann es bei Lichteinfall unter großen Winkeln auf den Grenzübergang zwischen Linsenmaterial und Kammerwasser des Auges zu totaler innerer Reflexion (auch "total internal reflexion" - TIR - genannt) kommen. Dieser Effekt tritt vor allem bei einem schrägen Einfall von Licht ins Auge auf und führte zu störenden Reflexen auf die Retina. Die reflektierenden Grenzflächen befinden sich dabei häufig außerhalb des Bereiches der Optik der künstlichen Augenlinse, die für eine Abbildung auf die Retina ausgelegt ist.

Durch die erfindungsgemäße Wahl der Brechungsindizes werden Dysphotopsien vermieden, da schräg einfallendes Licht - trotz totaler innerer Reflexion - nicht an scharfen Kanten gestreut werden kann. Dieser Vorteil kommt insbesondere bei künstlichen Augenlinsen zum Tragen, die ein erstes Linsenmaterial mit einem hohen ersten Brechungsindex aufweisen. Die Vermeidung von Dysphotopsien ist besonders effizient, wenn gar keine Kante in der Schnittebene im Bereich der festen Verbindung vorhanden ist.

In einer besonders vorteilhaften Ausgestaltung der künstlichen Augenlinse weicht der zweite Brechungsindex vom Brechungsindex von Kammerwasser oder Wasser um weniger als 0.05 ab, bevorzugt weniger als 0.01, besonders bevorzugt weniger als 0.003. Die Brechungsindizes von Wasser und Kammerwasser sind annähernd gleich.

Beispielhaft für ein solches zweites Linsenmaterial sei hier das Polymer "BIO-133" der Firma *MY Polymers Ltd.* genannt, das bei einer Wellenlänge von 589 nm einen Brechungsindex von 1.334 aufweist.

Ist der zweite Brechungsindex des zweiten Linsenmaterials, das dem Berandungselement zugeordnet ist, nahe am Brechungsindex vom Kammerwasser eines Auges, so treten bei einem Grenzübergang von Licht zwischen dem Berandungselement und dem Kammerwasser entsprechend der Fresnelschen Formel nur minimale Reflexionen auf. Auf diese Weise werden ungewollte Lichtreflexe, die an der scharfen Kante des Berandungselements hervorgerufen werden könnten (soweit Licht über den Grenzübergang zwischen Optikelement und Berandungselement überhaupt in dieses eindringen kann), weiter verringert.

Gemäß einer weiteren Ausgestaltung der künstlichen Augenlinse weist der zweite Brechungsindex einen Gradienten auf. Die Richtung des Gradienten erstreckt sich dabei innerhalb des Berandungselements von der festen Verbindung zum Optikelement hin zu einer Außenfläche des Berandungselement, die im implantierten Zustand der künstlichen Augenlinse in Kontakt mit dem Kammerwasser des Auges treten kann. Da der erste Brechungsindex des Optikelements typischerweise größer ist als der Brechungsindex vom Kammerwasser, ist auch der zweite Brechungsindex (entlang des Gradienten) auf der der festen Verbindung zugewandten Seite größer als der zweite Brechungsindex auf der der Außenfläche des Berandungselements zugewandten Seite. Bevorzugt weist der zweite Brechungsindex auf der der festen Verbindung zum Optikelement zugewandten Seite einen Wert auf, der vom ersten Brechungsindex des Optikelements um weniger als 0.05 abweicht, vorzugsweise um weniger als 0.03, besonders bevorzugt um weniger als 0.01. Bevorzugt ist der zweite Brechungsindex hier nicht größer als der erste Brechungsindex des Optikelements. Zusätzlich oder alternativ weist der zweite Brechungsindex auf der der Außenseite (Außenfläche) zugewandten Seite einen Wert auf, der vom Brechungsindex von Kammerwasser (oder Wasser) um weniger als 0.05 abweicht, vorzugsweise um weniger als 0.03, besonders bevorzugt um weniger als 0.01. Bevorzugt ist der zweite Brechungsindex hier (an der Außenfläche) nicht kleiner als der Brechungsindex von Kammerwasser (oder Wasser).

Der erfindungsgemäße Brechungsindex-Gradient des zweiten Linsenmaterials führt dazu, dass ungewollte Lichtreflexe an der Grenzfläche zwischen Optikelement und Berandungselement und/oder an der Grenzfläche zwischen Berandungselement und Kammerwasser weiter reduziert werden.

Eine Variante der Erfindung betrifft eine künstliche Augenlinse für eine Implantation in einem Auge. Die Variante der künstlichen Augenlinse umfasst ein Optikelement, das ein erstes Linsenmaterial mit einer ersten Absorption aufweist. Bei dem Linsenmaterial kann es sich beispielsweise um Acryl, Silicon oder Hydrogel handeln oder die genannten Materialien aufweisen. Das Linsenmaterial kann auch aus Polymethylmethacrylat (PMMA) bestehen (oder dieses aufweisen) oder andere Materialien aufweisen, die in der Medizintechnik üblich sind, biokompatibel sind und eine hinreichend hohe Transmission (also geringe Absorption) aufweisen. Dabei kann die Absorption über die Wellenlängen variieren, um beispielsweise mehr blaues Licht zu blockieren. Typischerweise ist die Transmission durch eine künstliche Augenlinse von beispielsweise 2 mm Dicke über 80% des sichtbaren Spektralbereichs (400 nm bis 750 nm) größer als 90%; die Absorption beträgt also hier typischerweise weniger als 10%. Ein Brechungsindex des ersten Linsenmaterials ist typischerweise größer als der von Wasser oder Kammerwasser (etwa 1.33); typischerweise ist er größer als 1.4.

Das Optikelement weist weiterhin eine Vorderseite, eine Rückseite und eine optische Hauptachse auf. Dabei ist die Vorderseite des Optikelement im implantierten Zustand der Kornea des Auges zugewandt und die Rückseite des Optikelement ist der Netzhaut (Retina) zugewandt. Vorderseite und Rückseite sind als optische Flächen ausgeformt. Die Vorderseite und/oder die Rückseite können sphärisch oder asphärisch ausgeformt sein. Sie können weiterhin die Form einer Freiformfläche annehmen, d.h. sie können beispielsweise über ein Polynom oder stückweise über Polynome beschrieben sein. Die Vorderseite und/oder die Rückseite können zusätzlich diffraktive optische Strukturen aufweisen, um beispielsweise mehr als einen Brechkraft bereitzustellen. Die Vorderseite und die Rückseite der Variante der künstlichen Augenlinse sind für die optischen Abbildungseigenschaften verantwortlich. Licht kann auf der Vorderseite in die Augenlinse eindringen und diese an der Rückseite wieder verlassen. Die optische Hauptachse steht senkrecht auf einer gedachten Ebene, die sich zwischen der Vorderseite und der Rückseite der Augenlinse befindet. Die optische Hauptachse durchstößt das Optikelement typischerweise in den Scheitelpunkten der Vorderseite und der Rückseite.

Weiterhin umfasst das Optikelement eine optische Zone, wobei die optische Zone zum Führen von Licht auf die Netzhaut des Auges ausgebildet ist. Bei der optischen Zone handelt es sich um ein Teilvolumen des Optikelements. Ein Teil der Vorderseite und ein Teil der Rückseite begrenzen die optische Zone. Im implantierten Zustand der Variante der künstlichen Augenlinse kann Licht durch die Vorderseite in die optische Zone eindringen und diese auf der Rückseite des Optikelements wieder in Richtung Netzhaut verlassen. Dabei wird das Licht abbildend geführt. Derjenige Teil des Optikelements, der nicht zur optischen Zone gehört, dient im implantierten Zustand der künstlichen Augenlinse nicht einer abbildenden Lichtführung.

Die Variante der künstliche Augenlinse umfasst weiterhin ein Berandungselement, die mit dem Optikelement außerhalb der optischen Zone eine feste Verbindung aufweist. Das Berandungselement beeinträchtigt somit die abbildende Lichtführung der Variante der künstlichen Augenlinse nicht. Dabei weist das Berandungselement eine scharfe Kante in einer Schnittebene auf, die die optische Hauptachse umfasst. Eine scharfe Kante liegt vor, wenn der in der Schnittebene innerhalb des Berandungselements gemessene Winkel an der Kante kleiner oder gleich 100° beträgt, bevorzugt kleiner oder gleich 90° (rechter Winkel). Die scharfe Kante des Berandungselements befindet sich in der Schnittebene außerhalb der Verbindung des Berandungselements mit dem Optikelement. Die scharfe Kante der erfindungsgemäßen Variante der Augenlinse behindert ein Zellwachstum über diese scharfe Kante hinaus in Richtung der optischen Zone und verringert somit das Risiko eines Nachstars.

Erfindungsgemäß weist das Optikelement im Bereich der festen Verbindung in der Schnittebene keine scharfe Kante auf. Betrachtet man in der Schnittebene also den Bereich des Optikelement, an dem diese die feste Verbindung mit dem Berandungselement aufweist, so gibt es dort keine Kante, die einen Winkel (gemessen innerhalb des Optikelements) von 100° oder weniger aufweist, bzw. von 90° oder weniger. Bevorzugt weisen alle Kanten in diesem Bereich Winkel auf, die größer als 110° sind, besonders bevorzugt größer als 130°. Besonders bevorzugt weist das Optikelement in diesem Bereich keine Kante auf; es treten keine Unstetigkeiten in der Krümmung auf.

Weiterhin weist das Berandungselement der erfindungsgemäßen Variante der künstlichen Augenlinse ein zweites Linsenmaterial mit einer zweiten Absorption auf. Dabei ist die zweite Absorption von der ersten Absorption verschieden. Die Absorption eines Materials kann durch einen Absorptionskoeffizienten beschrieben werden; dazu lässt sich das Lambert-Beer'sche Gesetz für eine gegebene Dicke des durchstrahlten Materials verwenden. Ein erster Absorptionskoeffizient des ersten Linsenmaterials ist also von einem zweiten Absorptionskoeffizient des zweiten Linsenmaterials verschieden. Vorzugsweise unterscheiden sich die Absorptionskoeffizienten derart, dass für eine Materialdicke von 0.5 mm die Transmission durch das zweite Linsenmaterial maximal 60% der Transmission durch das erste Linsenmaterial entspricht, bevorzugt maximal 20%, besonders bevorzugt maximal 5%. Dies gilt vorzugsweise über einen Bereich von mindestens 80% des sichtbaren Spektralbereichs (400 nm bis 750 nm).

Durch die unterschiedlichen Absorptionen des ersten Linsenmaterials des Optikelements und des zweiten Linsenmaterials des Berandungselements ergibt sich beim Übergang zwischen den beiden Materialien an deren fester Verbindung, dass kein Licht an einer scharfen Kante gestreut werden kann. Vielmehr kann Licht am Übergang zum Berandungselement bzw. innerhalb des Berandungselements absorbiert werden. Auf diese Weise können auch in dieser Variante der Erfindung sowohl ungewollte Lichtreflexe und Dysphotopsien vermieden als auch gleichzeitig aufgrund der scharfen Kante des Berandungselements außerhalb der festen Verbindung ein Zellwachstum behindert werden.

In einer bevorzugten Ausgestaltung der Variante der künstlichen Augenlinse weist diese zusätzlich Merkmale der zuerst ausgeführten künstlichen Augenlinse (sowie deren Ausgestaltungen) auf, deren erstes und zweites Linsenmaterial voneinander abweichende Brechungsindizes aufweisen. Dadurch lassen sich ungewollte Reflexe besonders effizient unterdrücken.

Gemäß einer bevorzugten Ausgestaltung der künstliche Augenlinse - sowohl nach einer der zuerst ausgeführten Ausgestaltungen als auch nach der Variante - weist die scharfe Kante des Brandungselements von der optischen Hauptachse einen geringeren Abstand auf als diejenigen Teile des Brandungselements und/oder Optikelements, die den größten Abstand von der optischen Hauptachse aufweisen. Typischerweise verwachsen nach der Implantation diejenigen Teile der künstlichen Augenlinse, die einen maximalen Abstand von der optischen Hauptachse aufweisen, mit dem Gewebe des Auges. Eine scharfe Kante im Berandungselement, das von der optischen Hauptachse einen geringeren Abstand aufweist, ist somit besonders geeignet, eine Zellwachstum zu verhindern.

Gemäß einer besonders bevorzugten Ausgestaltung der künstliche Augenlinse - sowohl nach einer der zuerst ausgeführten Ausgestaltungen als auch nach der Variante - umschließt das Berandungselement die Vorderseite und/oder die Rückseite des Optikelements im Wesentlichen vollständig. Dabei ist unter "im Wesentlichen vollständig" zu verstehen, dass das Berandungselement gegenüber der optischen Hauptachse mindestens 80% der Winkel abdeckt, bevorzugt mindestens 95%, besonders bevorzugt mindestens 99%. Das Berandungselement kann die Vorderseite und/oder die Rückseite auch vollständig umschließen. Dazu kann das Berandungselement ringförmig ausgestaltet sein.

Gemäß einer bevorzugten Weiterbildung der künstliche Augenlinse weist das Berandungselement für im Wesentlichen jede Schnittebene, die die optische Hauptachse umfasst, eine scharfe Kante auf. Zusätzlich oder alternativ weist das Optikelement im Bereich der festen Verbindung für im Wesentlichen jede Schnittebene (die die optische Hauptachse umfasst) keine scharfe Kante auf. Dabei sind unter "im Wesentlichen jeder Schnittebene" mindestens 80% aller möglicher Schnittebenen (die die optische Hauptachse umfassen) zu verstehen, bevorzugt mindestens 95%, besonders bevorzugt mindestens 99%.

Weist das Berandungselement für im Wesentlichen jede Schnittebene eine scharfe Kante auf, so wird ein Zellwachstum in Richtung der optischen Zone des Optikelements besonders gut unterbunden.

Bevorzugt weist die scharfe Kante des Brandungselements für im Wesentlichen jede Schnittebene einen geringeren Abstand von der optischen Hauptachse auf als diejenigen Teile des Brandungselements und/oder Optikelements, die den größten Abstand von der optischen Hauptachse aufweisen.

Weist das Optikelement für im Wesentlichen jede Schnittebene keine scharfe Kante auf, so werden ungewollte Lichtreflexe besonders effizient vermieden.

In einer weiteren bevorzugten Ausgestaltung der künstliche Augenlinse - sowohl nach einer der zuerst ausgeführten Ausgestaltungen als auch nach der Variante - umfasst diese weiterhin eine Haptik, die dazu ausgebildet ist, die künstliche Augenlinse im implantierten Zustand im Auge zu fixieren, wobei die Haptik einen Brechungsindex aufweist, der vom Brechungsindex von Kammerwasser (oder Wasser) um weniger als 0.01 abweicht, bevorzugt weniger als 0.003, besonders bevorzugt weniger als 0.001.

Die Haptik ist dazu ausgebildet, im Auge fixiert zu werden, vorzugsweise im Kapselsack oder am Sulcus Ciliaris. Nach einer Implantation der künstlichen Augenlinse ins Auge und der Wundheilung wächst die Haptik ins Gewebe des Auges ein.

Die Haptik der künstlichen Augenlinse kann ein Material aufweisen, das vom ersten Linsenmaterial und zweiten Linsenmaterial und deren jeweiligen Brechungsindizes verschieden ist; die Haptik ist dann weder Teil des Rahmenelements noch des Optikelements. Vorzugweise ist die Haptik Teil des Rahmenelements und weist das zweite Linsenmaterial oder weist ein Linsenmaterial mit dem zweiten Brechungsindex auf.

Durch eine Haptik, deren Brechungsindex nahe am Brechungsindex vom Kammerwasser ist, werden ungewollte Lichtreflexe an den Grenzübergängen zwischen Haptik und Kammerwasser weiter vermindert.

Vorzugsweise weist die Haptik einen Marker auf, der es erlaubt, die Haptik während der Implantation der künstlichen Augenlinse zu erkennen. Die ist deshalb vorteilhaft, da sich die Haptik aufgrund der fast identischen Brechungsindizes von Haptik und Kammerwasser im sichtbaren Licht kaum vom Kammerwasser abhebt. Die Markierung kann derart ausgestaltet sein, dass sie einen Teil des sichtbaren Lichtes absorbiert; so kann der Marker beispielsweise als bläulich gefärbt erscheinen. Der Marker kann auch so ausgestaltet sein, dass er beispielsweise im infraroten (IR) Spektralbereich sichtbar ist und sich so vom Kammerwasser abhebt; in diesem Fall erfolgt die Implantation vorzugsweise unter Verwendung einer IR-Beleuchtung und einer Kamera, die für IR-Licht sensitiv ist. Der Marker kann auch einen Fluoreszenzfarbstoff umfassen. Vorzugsweise ist der Marker so ausgestaltet, dass er sich nach der Implantation ins Auge auflöst. Weiterhin kann der Marker derart ausgestaltet sein, dass ein Teil der Oberfläche der Haptik aufgeraut ist.

Vorzugsweise ist die scharfe Kante des Berandungselements derart ausgestaltet und/oder angeordnet, dass sie optisch nicht-abbildend ist. Als Teil einer diffraktiven Struktur kann eine Kante Licht in eine Beugungsordnung ablenken und somit abbildend sein. Weist ein Berandungselement mehr als eine scharfe Kante auf (oder weist das Optikelement eine diffraktive Struktur auf), so weisen diese scharfen Kanten erfindungsgemäß einen Abstand voneinander auf (bzw. weisen gegenüber der diffraktiven Struktur des Optikelements einen Abstand auf), der für eine kohärente Überlagerung ungeeignet ist. Auf diese Weise wird verhindert, dass Licht, das auf die scharfe Kante fällt, abbildend in Richtung der Retina des Auges gelenkt werden kann und dort ungewollte Lichtreflexe und Dysphotopsien verursacht.

Ein weiterer Aspekt der Erfindung betrifft ein Verfahren zur Herstellung einer künstlichen Augenlinse nach einer der Ausgestaltungen und Varianten, wie sie oben beschrieben wurden. Das Verfahren umfasst eine Fertigung des Optikelements. Die Herstellung des Optikelements kann dabei nach einem bekannten Fertigungsverfahren wie Gießen erfolgen, bei dem erwärmtes, flüssiges Linsenmaterial in eine Form gegossen und nach dem Erkalten aus der Form herausgelöst wird. Die Fertigung des Optikelements kann auch über ein abtragendes Verfahren erfolgen, bei dem beispielsweis von einem rotierenden Rohling Material von einem Diamantwerkzeug abgetragen wird. Zusätzlich können die optischen Oberflächen des Optikelements poliert werden. Das Optikelement wird aus dem ersten Linsenmaterial mit dem ersten Brechungsindex bzw. der ersten Absorption gefertigt.

Das Herstellungsverfahren umfasst weiterhin eine Fertigung des Berandungselements. Auch hier kann die Fertigung nach einem wie oben beschriebenen, bekannten Verfahren erfolgen. In diesem Fertigungsschritt wird die scharfe Kante hergestellt. Die Fertigung des Optikelements und des Berandungselements erfolgt jeweils getrennt voneinander. Das Berandungselement wird aus dem zweiten Linsenmaterial mit dem zweiten Brechungsindex bzw. der zweiten Absorption gefertigt.

Weiterhin umfasst das Herstellungsverfahren ein Verbinden des Optikelements mit dem Berandungselement. Auf diese Weise wird die feste Verbindung zwischen den beiden Elementen erzeugt. Das Verbinden kann beispielsweise durch Kleben oder Schweißen erfolgen. Im Falle von Kleben weist der Klebstoff vorteilhat einen Brechungsindex auf, der dem des Optikelements oder des Berandungselements entspricht (oder zwischen diesen beiden Brechungsindizes liegt). Im Falle von Schweißen und Kleben werden durch den Verbindungsvorgang die optischen Eigenschaften der künstlichen Augenlinse nicht beeinträchtigt, da die Verbindung zwischen Optik- und Berandungselement außerhalb der optischen Zone erfolgt.

Werden Optikelement und Berandungselement getrennt gefertigt, so können dadurch Zeit und/oder Kosten eingespart werden: So können beispielsweise bei Herstellung mit einem abtragenden Verfahren für die beiden Elemente unterschiedliche Werkzeuge für den Abtrag verwendet werden, da die Ausarbeitung einer scharfen Kante am Berandungselement andere Werkzeuge erfordert als sie für das Optikelemente, das keine scharfe Kante aufweist, benötigt werden. So kann die Zeit für einen Wechsel des Werkzeugs eingespart werden. Im Fall der Herstellung mittels Gießens oder Spritzens können die Formen mit unterschiedlichen Toleranzen hergestellt werden und/oder die Herstellung kann mit unterschiedlichen Prozessparametern durchgeführt werden, die jeweils auf die Ausformung der scharfen Kante bzw. auf die optischen Flächen ausgerichtet sind.

Gemäß einem alternativen Verfahren zur Herstellung einer künstlichen Augenlinse nach einer der Ausgestaltungen und Varianten, wie sie oben beschrieben wurden, umfasst dieses eine Fertigung des Optikelements. Die Herstellung des Optikelements kann dabei nach einem der oben beschriebenen Verfahren erfolgen. Das Optikelement wird aus dem ersten Linsenmaterial mit dem ersten Brechungsindex bzw. der ersten Absorption gefertigt.

Weiterhin umfasst das Verfahren ein Einbringen des Optikelements in eine Gussform. Dabei ist die Gussform (auch "mold" genannt) so ausgestaltet, dass das Optikelement dort gehalten wird und gleichzeitig freies Volumen innerhalb der Gussform vorhanden ist, das für eine Ausformung des Berandungselements geeignet ist. Schließlich umfasst das Verfahren ein Überspritzen des Optikelements in der Gussform mit dem zweiten Linsenmaterial zur Erzeugung des Berandungselements. Dieser Prozessschritt wird auch als "overmolding" bezeichnet. Das Überspritzen erfolgt mit dem zweiten Linsenmaterial, das den zweiten Brechungsindex bzw. die zweite Absorption aufweist. In diesem Prozessschritt erfolgt die Fertigung der scharfen Kante des Berandungselements.

In einem weiteren alternativen Verfahren zur Herstellung einer künstlichen Augenlinse nach einer der Ausgestaltungen und Varianten, wie sie oben beschrieben wurden, umfasst dieses eine Bereitstellung eines Linsenrohlings. Weiterhin umfasst das Verfahren eine Herausarbeitung der Form der künstlichen Augenlinse. **In** diesem Prozessschritt erhalten das Optikelement, das Berandungselement sowie eine ggf. vorhandene Haptik ihre geometrische Form. Die optischen Flächen des Optikelement werden hier erzeugt. Das Berandungselement erhält eine scharfe Kante.

Weiterhin umfasst das Verfahren eine Beaufschlagung des Optikelements oder des Berandungselements mit Licht derart, dass der Brechungsindex oder die Absorption des ersten oder zweiten Linsenmaterials derart verändert wird, dass der erste Brechungsindex bzw. die erste Absorption vom zweiten Brechungsindex bzw. von der zweiten Absorption verschieden ist. Durch die Beaufschlagung mit Licht können beispielsweise chemische Bindungen im Linsenmaterial aufgebrochen werden, die für einen hohen Brechungsindex verantwortlich sind. Durch das Aufbrechen der Bindungen wird der Brechungsindex verringert. Alternativ kann durch die Beaufschlagung mit Licht eine Struktur von Nanoporen im Linsenmaterial erzeugt werden, es können sogenannte Vakuolen eingeschossen werden. Diese Nanoporen erlauben das Aufnehmen von Wasser (Kammerwasser) und verändern somit (nach Implantation ins Auge) den Brechungsindex, da sich dieser dem Brechungsindex von Wasser annähert.

Vor der Beaufschlagung mit Licht können die Brechungsindizes bzw. die Absorptionen der Linsenmaterialien von Optikelement und Berandungselement noch gleich sein. Erst durch den genannten Verfahrensschritt wird der Unterschied zwischen den Brechungsindizes bzw. den Absorptionen erzeugt.

Bevorzugt werden vor der Beaufschlagung mit Licht Teile der künstlichen Augenlinse mit einer Schutzschicht versehen, die beispielsweise lichtundurchlässig ist. Auf diese Weise kann die Licht-induzierte Anpassung von Brechungsindex bzw. Absorption gezielt auf bestimmte Teile der künstlichen Augenlinse beschränkt werden. Beispielsweise kann das Optikelement mit einer Schutzschicht versehen werden, so dass durch eine Beaufschlagung der künstlichen Augenlinse mit Licht lediglich die Materialeigenschaften des Berandungselements (und ggf. der Haptik) verändert werden.

Gemäß einem weiteren alternativen Verfahren zur Herstellung einer künstlichen Augenlinse nach einer der Ausgestaltungen und Varianten, wie sie oben beschrieben wurden, umfasst dieses eine Bereitstellung eines Linsenrohlings. Weiterhin umfasst das Verfahren eine Herausarbeitung der Form der künstlichen Augenlinse. In diesem Prozessschritt erhalten das Optikelement, das Berandungselement sowie eine ggf. vorhandene Haptik ihre geometrische Form. Die optischen Flächen des Optikelement werden hier erzeugt. Das Berandungselement erhält eine scharfe Kante.

Weiterhin umfasst das Verfahren eine Beaufschlagung des Optikelements oder des Berandungselements mit einer Chemikalie derart, dass der Brechungsindex oder die Absorption des ersten oder zweiten Linsenmaterials derart verändert wird, dass der erste Brechungsindex bzw. die erste Absorption vom zweiten Brechungsindex bzw. von der zweiten Absorption verschieden ist. Bei der Chemikalie kann es sich um eine chemische Lösung handeln, in die die Augenlinse eingetaucht wird. Es kann sich auch um ein Gas oder Dampf handelt, denen die Augenlinse ausgesetzt wird. Durch die Beaufschlagung mit der Chemikalie können beispielsweise chemische Bindungen im Linsenmaterial aufgebrochen werden, die für einen hohen Brechungsindex verantwortlich sind. Durch das Aufbrechen der Bindungen wird der Brechungsindex verringert. Die Chemikalie kann beispielsweise Fluor umfassen, das sich in das Linsenmaterial einbindet und so den Brechungsindex senkt. Alternativ kann durch die Beaufschlagung mit der Chemikalie eine Struktur von Nanoporen im Linsenmaterial erzeugt werden. Diese Nanoporen erlauben das Aufnehmen von Wasser (Kammerwasser) und verändern somit (nach Implantation ins Auge) den Brechungsindex, da sich dieser dem Brechungsindex von Wasser annähert.

Auch in diesem alternativen Herstellungsverfahren können vor der Beaufschlagung mit der Chemikalie die Brechungsindizes bzw. die Absorptionen der Linsenmaterialien von Optikelement und Berandungselement noch gleich sein. Erst durch den genannten Verfahrensschritt wird der Unterschied zwischen den Brechungsindizes bzw. den Absorptionen erzeugt.

Bevorzugt werden vor der Beaufschlagung mit der Chemikalie Teile der künstlichen Augenlinse mit einer Schutzschicht versehen, die die Wirkung der Chemikalie auf das Linsenmaterial unterbindet (oder zumindest hemmt). Auf diese Weise kann die chemisch-induzierte Anpassung von Brechungsindex bzw. Absorption gezielt auf bestimmte Teile der künstlichen Augenlinse beschränkt werden. Beispielsweise kann das Optikelement mit einer Schutzschicht versehen werden, so dass durch eine Beaufschlagung der künstlichen Augenlinse mit der Chemikalie lediglich die Materialeigenschaften des Berandungselements (und ggf. der Haptik) verändert werden.

Nachfolgend wird die Erfindung beispielsweise anhand der beigefügten Zeichnungen, die auch erfindungswesentliche Merkmale offenbaren, näher erläutert. Es zeigen:
Fig. 1 eine schematische Darstellung der simulierten Wirkung in einer Schnittebene für eine künstliche Augenlinse nach dem Stand der Technik nach Implantation in ein Auge;
Fig. 2 eine perspektivische Darstellung eines ersten Ausführungsbeispiels einer erfindungsgemäßen künstlichen Augenlinse;
Fig. 3 eine schematische Darstellung eines zweiten Ausführungsbeispiels einer erfindungsgemäßen künstlichen Augenlinse in einer Schnittebene;
Fig. 4 eine schematische Darstellung eines Ausschnitts einer dritten Ausführungsbeispiels einer künstlichen Augenlinse in einer Schnittebene;
Fig. 5 eine schematische Darstellung eines Ausschnitts einer vierten Ausführungsbeispiels einer künstlichen Augenlinse in einer Schnittebene;
Fig. 6a und 6b schematische Darstellungen der Herstellung einer erfindungsgemäßen künstlichen Augenlinse nach einer ersten Variante;
Fig. 7 eine schematische Darstellung der Herstellung einer erfindungsgemäßen künstlichen Augenlinse nach einer zweiten Variante;
Fig. 8a bis d schematische Darstellungen der Herstellung einer erfindungsgemäßen künstlichen Augenlinse nach einer dritten Variante.

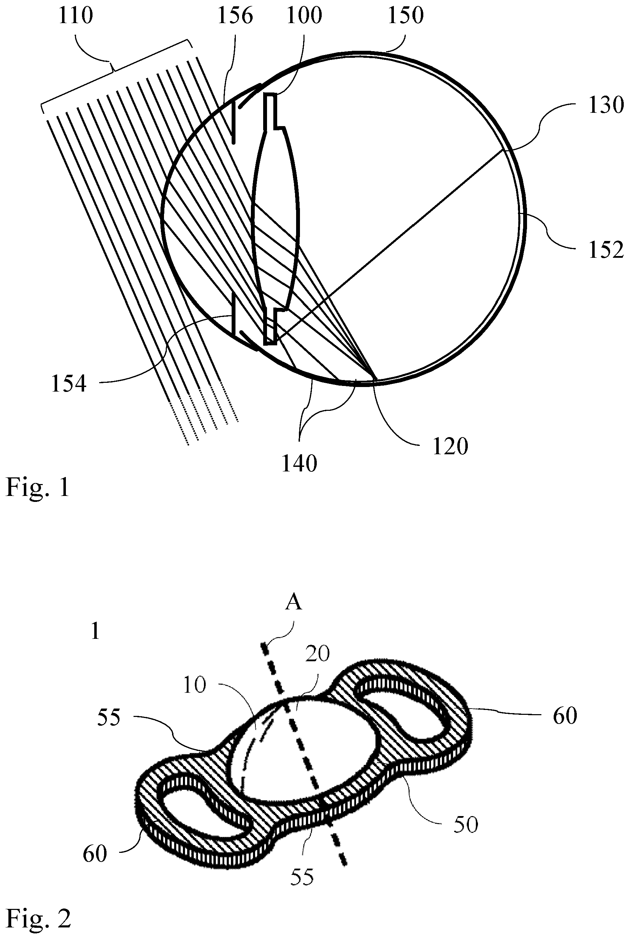

In Fig. 1 ist eine schematische Darstellung (in einer Schnittebene) einer Strahlenverfolgungs-Simulation (auch "raytracing" genannt) der Wirkung einer künstlichen Augenlinse 100 nach dem Stand der Technik nach Implantation in ein Auge 150 gezeigt. Ein schräg einfallendes, paralleles Lichtbündel 110 fällt auf die Kornea 156 des Auges 150. Ein Teil des Lichtbündels 110 wird von der Kornea 156 derart abgelenkt, dass es auf die Iris 154 fällt und dort absorbiert (oder reflektiert) wird und nicht weiter ins Auge 150 eindringt. Ein Großteil des durch die Kornea 156 abgelenkten Strahlbündels 110 fällt auf die künstliche Augenlinse 100 nach dem Stand der Technik. Licht, das auf den zentralen optisch wirksamen Teil der Augenlinse 100 fällt, wird entsprechend der optischen Wirkung der Augenlinse 100 auf die Retina 152 abgebildet; dort entsteht ein Fokus 120. Die Simulation zeigt weiterhin, dass Licht des Strahlbündels 110, das außerhalb des optisch wirksamen Teils der Augenlinse 100 - beispielsweise die Haptik oder den Rand des optisch wirksamen Teils - auftrifft, an dort vorhandenen Kanten, die ein Zellwachstum behindern, gestreut wird. Weiterhin kann es zu totaler innerer Reflexion kommen auf Grund des Brechungsindex-Sprungs zwischen der Augenlinse 100 und dem umgebenden Kammerwasser; dieser Effekt ist bei hochbrechenden Linsenmaterialien besonders gravierend. Beide Effekte führen dazu, dass Licht außerhalb des vorgesehenen Fokus 120 auf die Retina 152 fallen und dort positive Dysphotopsie 130 verursachen. Weiterhin treten negative Dysphotopsien 140 als Schatten auf. Diese Effekte führen also zu ungewollten Lichtreflexionen und somit zu einer Verschlechterung der Sehleistung bzw. Verringerung der Akzeptanz des Patienten für die künstliche Augenlinse 100.

Die gezeigten Simulationen wurden für einen Brechungsindex der künstlichen Augenlinse 100 nach dem Stand der Technik von 1.45 und für einen Brechungsindex des umgebenden Kammerwassers von 1.33. durchgeführt.

In Fig. 2 ist eine perspektivische Darstellung eines ersten Ausführungsbeispiels einer erfindungsgemäßen Augenlinse 1 gezeigt, die für eine Implantation im Kapselsack eines Auges ausgebildet ist. Die Augenlinse 1 umfasst ein Optikelement 10 mit einer Vorderseite 20, die im implantierten Zustand der künstlichen Augenlinse 1 der Kornea zugewandt ist. Die Rückseite des Optikelements 10, die im implantierten Zustand der Linse der Retina zugewandt ist, ist in der perspektivischen Darstellung nicht zu sehen. Das Optikelement 10 weist eine optische Hauptachse A auf. Es weist zudem ein erstes Linsenmaterial auf; in dieser und den nachfolgenden Figuren ist das erste Linsenmaterial ohne Schraffur dargestellt. Das Optikelement 10 weist keine Kante auf - und somit auch keine scharfe Kante.

Die künstliche Augenlinse 1 umfasst weiterhin ein Berandungselement 50. Das Berandungselement 50 umschließt die Vorderseite 20 und die Rückseite des Optikelements 10 vollständig. Im gezeigten Ausführungsbeispiel ist ein Teil des Berandungselements 50 als Haptik 60 ausgeformt. Es handelt sich hier um eine sogenannte Plattenhaptik. Alternative Haptiken wie beispielsweise sogenannte C-Loops sind ebenfalls denkbar.

Das Berandungselement 50 weist ein zweites Linsenmaterial auf, das in dieser und den nachfolgenden Figuren mittels Schraffur dargestellt ist. Im hier dargestellten Ausführungsbeispiel unterscheiden sich die beiden Linsenmaterialien in ihren Brechungsindizes. Die Materialien könnten sich aber auch zusätzlich oder alternativ in ihrer Absorption unterscheiden. Aufgrund der unterschiedlichen Brechungsindizes der Linsenmaterialien und fehlender Kanten des Optikelements 10 können ungewollte Lichtreflexe besonders effizient unterbunden werden.

Das Berandungselement 50 weist weiterhin scharfe Kanten 55 (mit Winkeln von 90° innerhalb des Berandungselements 50 gemessen) auf. Diese Kanten 55 behindern ein Zellwachstum über die Kanten 55 hinweg und vermindern somit das Risiko eines Nachstars. Im gezeigten Ausführungsbeispiel sind sowohl auf der der Kornea zugewandten Seite des Berandungselements 50 als auch auf der der Retina zugewandten Seite des Berandungselements 50 scharfe Kanten 55 vorhanden. Die Kanten 55 erstrecken sich ebenfalls auf die Haptiken 60.

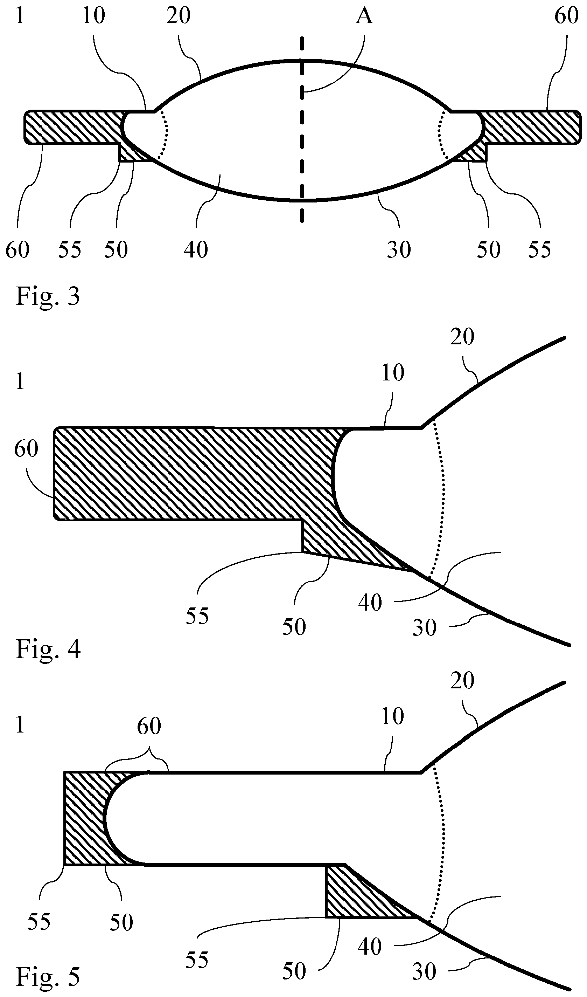

In Fig. 3 ist eine schematische Darstellung eines zweiten Ausführungsbeispiels einer künstlichen Augenlinse 1 in einer Schnittebene gezeigt. Die Schnittebene umfasst dabei die optische Hauptachse A. Die Schnittebene ist so ausgerichtet, dass sie an beiden Enden der künstlichen Augenlinse 1 die Haptiken 60 schneidet. Das Optikelement 10 der künstlichen Augenlinse 1 weist eine Vorderseite 20 und eine Rückseite 30 sowie ein erstes Linsenmaterial mit einem ersten Brechungsindex auf. Im implantierten Zustand der Augenlinse 1 kann Licht auf der Vorderseite 20 in das Optikelement 10 eindringen und dieses an der Rückseite 30 wieder verlassen. Die Krümmungen von Vorderseite 20 und Rückseite 30 sowie der erste Brechungsindex des ersten Linsenmaterials bestimmen die Brechkraft der künstlichen Augenlinse 1. Die zum Führen von Licht auf die Netzhaut des Auges ausgebildete optische Zone 40 wird durch die Vorderseite 20, die Rückseite 30 sowie seitlich durch die gepunktet eingezeichneten Linien markiert. Außerhalb der optischen Zone 40 ist das Optikelement 10 mit dem Berandungselement 50 fest verbunden. Auch wenn Figur 3 lediglich eine Schnittebene zeigt, so umschließt das Berandungselement 50 in diesem Ausführungsbeispiel das Optikelement 10 vollständig. Die Haptiken 60 sind Teil des Berandungselements 50. Das Berandungselement 50 weist in der gezeigten Schnittebene eine scharfe Kante 55 auf. Die scharfe Kante 55 befindet sich dabei auf der der Retina zugewandten Seite der künstlichen Augenlinse 1. Der im Berandungselement 50 gemessene Winkel der Kante 55 beträgt 90°. Die scharfe Kante 55 bewirkt, dass ein Zellwachstum, das aus Richtung der Haptiken 60 kommt, an dieser Kante 55 unterbunden wird, so dass das Risiko von Nachstar verringert wird.

Während das Optikelement 10 ein erstes Linsenmaterial (eingezeichnet ohne Schraffierung) mit einem ersten Brechungsindex aufweist, umfasst das Berandungselement 50 ein zweite Linsenmaterial (eingezeichnet mit schräger Schraffur) mit einem zweiten Brechungsindex, der vom ersten Brechungsindex verschieden ist. Im Bereich der festen Verbindung zwischen Optikelement 10 und Berandungselement 50 weist das Optikelement 10 keine Kante auf. Auf diese Weise können erfindungsgemäß Dysphotopsien - auch bei schrägem Lichteinfall - vermindert werden.

Im gezeigten Ausführungsbeispiel weist die scharfe Kante 55 von der optischen Hauptachse A einen geringeren Abstand auf als diejenigen Teile des Brandungselements 50, die den größten Abstand von der optischen Hauptachse aufweisen, in diesem Beispiel weist die Haptik 60, die Teil des Brandungselements 50 ist, an seiner äußersten Kante den größten Abstand von der optischen Hauptachse A auf. Ist nach Implantation der Augenlinse 1 diese im Bereich der Haptik 60 mit dem Auge verwachsen, so behindert die scharfe Kante 55 besonders gut ein Zellwachstum, da die scharfe Kante 55 einen geringeren Abstand von der optischen Hauptachse A aufweist und somit in der Regel nicht ins Gewebe des Auges eingewachsen ist.

Fig. 4 zeigt eine schematische Darstellung eines Ausschnitts eines dritten Ausführungsbeispiels einer künstlichen Augenlinse 1 in einer Schnittebene. Bei dem Ausschnitt handelt es sich um einen Teil eines Schnittes durch die künstliche Augenlinse 1, der die optische Hauptachse (nicht eingezeichnet) enthält. Dabei beinhaltet der gezeigte Ausschnitt die Haptik 60, das Berandungselement 50 sowie einen Teil des Optikelements 10 (mit Vorderseite 20, Rückseite 30 und optischer Zone 40). Die Ausgestaltung des Optikelements entspricht der des zweiten Ausführungsbeispiels nach Fig. 3. Abweichend zum zweiten Ausführungsbeispiel von Fig. 3 weist hier die scharfe Kante 55 einen im Berandungselement 50 gemessenen Winkel von 100°. Auch ein solcher Winkel ist für eine Behinderung von Zellwachstum geeignet.

Auch in diesem Ausführungsbeispiel weist die scharfe Kante 55 von der optischen Hauptachse (nicht eingezeichnet) einen geringeren Abstand auf als diejenigen Teile des Brandungselements 50, die den größten Abstand von der optischen Hauptachse aufweisen (hier der Haptik 60).

In Fig. 5 ist eine schematische Darstellung eines Ausschnitts eines vierten Ausführungsbeispiels einer künstlichen Augenlinse 1 in einer Schnittebene gezeigt. Der Ausschnitt entspricht dem aus der Darstellung von Fig. 4. In diesem Ausführungsbeispiel erstreckt sich das Optikelement 10 (mit erstem Linsenmaterial und erstem Brechungsindex) außerhalb von Vorderseite 20 und Rückseite 30 weiter seitlich und bildet einen Teil der Haptik 60. Zusätzlich ist das Berandungselement 50 zweiteilig ausgestaltet. Ein erster Teil des Berandungselements 50 ist mit der Rückseite 30 des Optikelements verbunden. Das Berandungselement 50 weist hier eine scharfe Kante 55 auf, deren Winkel 90° beträgt. Ein zweiter Teil des Berandungselements 50 ist mit einem äußeren Rand des Teils der Haptik 60 vom Optikelement 10 verbunden. An beiden Verbindungen zwischen Optikelement 10 und Berandungselement 50 weist das Optikelement 10 keine scharfe Kante auf. Beide Teile des Berandungselements 50 weisen jedoch scharfe Kanten 55 auf. Auf diese Weise kann ein Zellwachstum sowohl nahe der optisch wirksamen Fläche (Rückseite 30) des Optikelements 10 als auch an der Haptik 60 verhindert werden.

Es sei angemerkt, dass eine scharfe Kante 55 im Bereich der Haptik 60 nicht auf eine zweiteilige Ausführung des Berandungselements 50 beschränkt. Vielmehr kann auch für die vorgenannten Ausführungsbeispiele mit einem einteiligen Berandungselement 50 dieses im Bereich der Haptik 60 eine scharfe Kante 55 aufweisen.

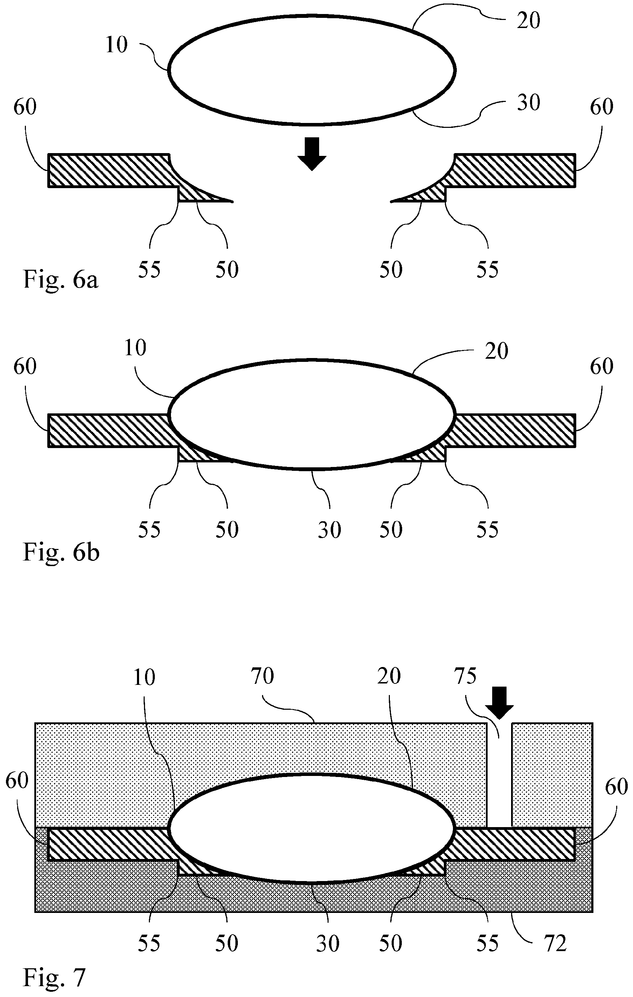

In Fig. 6a und 6b sind schematische Darstellungen der Herstellung einer erfindungsgemäßen künstlichen Augenlinse nach einer ersten Variante gezeigt. In diesem Verfahren werden das Optikelement 10 (mit Vorderseite 20, Rückseite 30 sowie der nicht eingezeichneten optischen Zone und optischer Hauptachse) und das Berandungselement 50 in zwei getrennten Schritten hergestellt. Nach ihrer jeweiligen Herstellung handelt es sich um zwei getrennte Teile. Die beiden genannten Elemente weisen unterschiedliche Linsenmaterialien mit voneinander abweichenden Brechungsindizes auf (Fig. 6a). In einem weiteren Herstellungsschritt werden Optikelement 10 und Berandungselement 50 dann fest verbunden (Fig. 6b). Im gezeigten Beispiel findet ein Kleben statt, wobei der Klebstoff nicht eingezeichnet ist.

Vorteil eines derartigen Verfahrens ist es, dass für die Herstellung von Optikelement 10 und Berandungselement 50 unterschiedliche Verfahren und Werkzeuge verwendet werden können, die an die Anforderungen der Elemente angepasst sind. Da das Optikelement 10 eine hohe Anforderung an eine Oberflächenrauigkeit stellt, jedoch häufig keine scharfen Kanten aufweist, können hier beispielsweise in einem Drehverfahren Diamantwerkzeuge mit anderen (größeren) Durchmesser verwendet werden als für das Berandungselement 50, das geringe Anforderungen an die Oberflächenrauigkeit stellt aber zur Verminderung von Zellwachstum eine scharfe Kante 55 aufweist.

Fig. 7 zeigt eine schematische Darstellung der Herstellung einer erfindungsgemäßen künstlichen Augenlinse nach einer zweiten Variante. Hier wird zunächst das Optikelement 10 (mit Vorderseite 20, Rückseite 30 sowie der nicht eingezeichneten optischen Zone und optischer Hauptachse) mit einem ersten Linsenmaterial und einem ersten Brechungsindex gefertigt. Dieses wir anschließend in eine Gussform 70, 72 (auch "mold" genannt) eingebracht, die im gezeigten Beispiel zweiteilig ausgestaltet ist. Die Gussform 70, 72 weist eine Öffnung 75 auf, über die ein zweites Linsenmaterial mit einem zweiten Brechungsindex eingefüllt werden kann (dargestellt durch soliden Pfeil). Dieser Prozess wird häufig als Überspritzen oder "overmolding" bezeichnet.

Auch über dieses Verfahren kann vorteilhaft das Optikelement 10 mit hoher Präzision gefertigt werden, während die Genauigkeitsanforderungen für das gegossenen Berandungselement 50 geringer sind - auch für die Ausarbeitung der scharfen Kante 55.

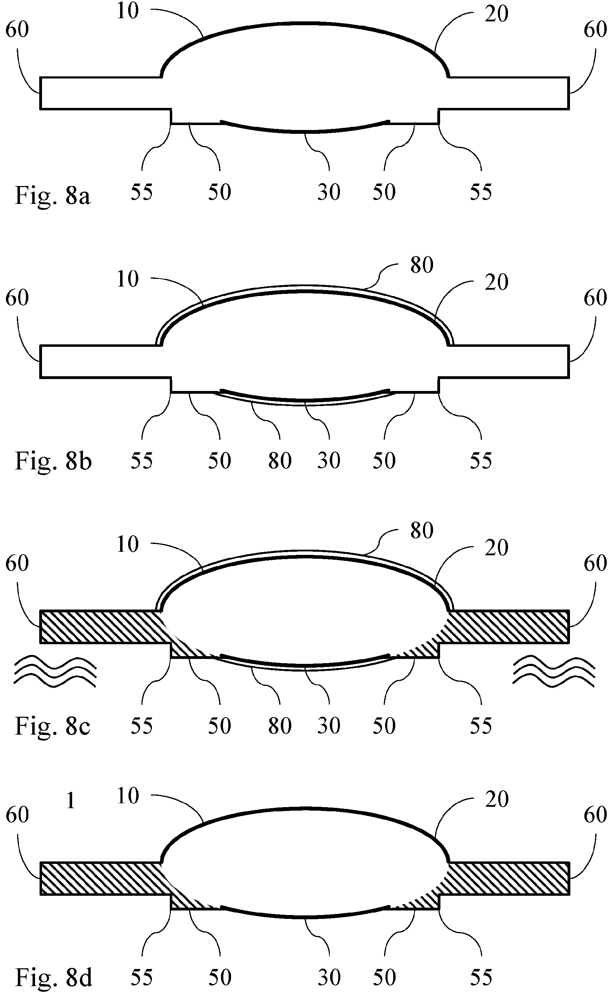

Fig. 8a bis d zeigen eine schematische Darstellung der Herstellungsschritte einer erfindungsgemäßen künstlichen Augenlinse nach einer dritten Variante. Zunächst wird hier aus einem Linsenrohling die Form der künstlichen Augenlinse herausgearbeitet. Optikelement 10 und Berandungselement 50 sowie die Haptiken 60 weisen danach ihre gewünschte Form auf (Fig. 8a). Im gezeigten Beispiel weist die künstlichen Augenlinse zu diesem Zeitpunkt des Herstellungsverfahrens das erste Linsenmaterial mit dem ersten Brechungsindex auf. Dies gilt auch für das Berandungselement 50 (dessen scharfe Kante 55 schon herausgearbeitet ist).

Im nächsten Schritt werden die Vorderseite 20 und die Rückseite 30 des Optikelements 10 mit einer Schutzschicht 80 versehen (siehe Fig. 8b). Anschließend wird die künstliche Augenlinse in ein chemisches Bad eingebracht; dies ist in Fig. 8c durch wellenförmige Symbole dargestellt. Durch das chemische Bad werden die Materialeigenschaftes des ersten Linsenmaterials derart geändert, dass eine Brechungsindex-Änderung erfolgt. Dieser Prozess findet überall dort statt, wo die Chemikalie des Bades mit der künstlichen Augenlinse in Kontakt tritt. Dieser Prozess ist beispielhaft dazu geeignet, einen Gradienten des zweiten Brechungsindex zu erzeugen. Aufgrund der Schutzschicht 80 findet dieser Prozess nicht im Bereich des Optikelements 10 statt, so dass dieses nach dem Bad unverändert das erste Linsenmaterial mit dem ersten Brechungsindex aufweist. Der Prozess erstreckt sich vielmehr auf das Berandungselement, das nun das zweite Linsenmaterial mit dem zweiten Brechungsindex aufweist (dargestellt durch die Schraffur).

In einem weiteren Schritt wird die Schutzschicht 80 entfernt. Fig. 8d zeigt nun eine fertiggestellte erfindungsgemäße künstliche Augenlinse 1. Optional kann anschließend die Form der künstlichen Augenlinse 1 in einem weiteren Prozessschritt nachgearbeitet werden, um beispielsweise eine geforderte optische Qualität der Vorderseite 20 und Rückseite 30 des Optikelements 10 sicherzustellen.

Das in den Fig. 8a bis d gezeigte Verfahren ist nicht auf die Verwendung eines chemischen Bades (Fig. 8c) beschränkt. Vielmehr können in diesem Schritt beispielsweise auch ein Gas oder Dampf verwendet werden, um die Materialeigenschaften zu verändern. Ebenfalls ist die Beaufschlagung mit Licht denkbar.

Es sei angemerkt, dass für die gezeigten Ausführungsbeispiele der künstlichen Augenlinse und für deren Herstellungsverfahren nicht nur unterschiedliche Brechungsindizes der Linsenmaterialien denkbar sind, sondern zusätzlich oder alternativ können die Linsenmaterialien auch unterschiedliche Absorptionen aufweisen.

Die vorstehend genannten und in verschiedenen Ausführungsbeispielen beschriebenen Merkmale der Erfindung sind dabei nicht nur in den angegebenen beispielhaften Kombinationen, sondern auch in anderen Kombinationen oder allein einsetzbar, ohne den Rahmen der vorliegenden Erfindung zu verlassen.

Eine auf Verfahrensmerkmale bezogene Beschreibung einer Vorrichtung gilt bezüglich dieser Merkmale analog für das entsprechende Verfahren, während Verfahrensmerkmale entsprechend funktionelle Merkmale der beschriebenen Vorrichtung darstellen.

## Patentansprüche

1. Künstliche Augenlinse (1) für eine Implantation in einem Auge, umfassend:
ein Optikelement (10) mit einer Vorderseite (20), einer Rückseite (30),
einer optischen Zone (40) und einer optischen Hauptachse (A), wobei
- die optische Zone (40) zum Führen von Licht auf eine Netzhaut des Auges ausgebildet ist,
und
ein Berandungselement (50), das mit dem Optikelement (10) außerhalb der optischen Zone (40) eine feste Verbindung aufweist, wobei
- das Berandungselement (50) eine scharfe Kante (55) in einer Schnittebene aufweist, die die optische Hauptachse (A) umfasst, wobei eine scharfe Kante (55) vorliegt, wenn der in der Schnittebene innerhalb des Berandungselements (50) gemessene Winkel an der Kante (55) kleiner oder gleich 100° beträgt, bevorzugt kleiner oder gleich 90°,
wobei das Optikelement (10) im Bereich der festen Verbindung in der Schnittebene keine scharfe Kante aufweist
**dadurch gekennzeichnet,**
- **dass** das Optikelement (10) ein erstes Linsenmaterial mit einem ersten Brechungsindex aufweist,
- **dass** das Berandungselement (50) ein zweites Linsenmaterial mit einem zweiten Brechungsindex aufweist, und
- **dass** der erste Brechungsindex größer ist als der zweite Brechungsindex.

2. Künstliche Augenlinse (1) nach Anspruch 1, wobei der zweite Brechungsindex vom Brechungsindex von Kammerwasser um weniger als 0.05 abweicht, bevorzugt weniger als 0.01, besonders bevorzugt weniger als 0.003.

3. Künstliche Augenlinse (1) nach einem der vorgenannten Ansprüche, wobei der zweite Brechungsindex einen Gradienten aufweist.

4. Künstliche Augenlinse (1) nach einem der vorgenannten Ansprüche, wobei das Berandungselement (50) die Vorderseite (20) und/oder die Rückseite (30) des Optikelements (10) im Wesentlichen vollständig umschließt.

5. Künstliche Augenlinse (1) nach Anspruch 4, wobei das Berandungselement (50) für im Wesentlichen jede Schnittebene eine scharfe Kante (55) aufweist und/oder dass das Optikelement (10) im Bereich der festen Verbindung für im Wesentlichen jede Schnittebene keine scharfe Kante aufweist.

6. Künstliche Augenlinse (1) nach einem der vorgenannten Ansprüche, wobei die Augenlinse (1) weiterhin eine Haptik (60) umfasst, die dazu ausgebildet ist, die Augenlinse (1) im implantierten Zustand im Auge zu fixieren, wobei die Haptik (60) einen Brechungsindex aufweist, der vom Brechungsindex von Kammerwasser um weniger als 0.01 abweicht, bevorzugt weniger als 0.003, besonders bevorzugt weniger als 0.001.

7. Verfahren zur Herstellung einer künstlichen Augenlinse (1) nach einem der Ansprüche 1 bis 6, wobei das Verfahren folgende Schritte umfasst:
- Fertigen des Optikelements (10), wobei das Optikelement (10) das erste Linsenmaterial mit dem ersten Brechungsindex aufweist,
- Fertigen des Berandungselements (50) sowie Fertigen der scharfen Kante (55) des Berandungselements (50), wobei das Berandungselements (50) das zweite Linsenmaterial mit dem zweiten Brechungsindex aufweist, wobei der erste Brechungsindex größer ist als der zweite Brechungsindex,
- Verbinden des Optikelements (10) mit dem Berandungselement (50).

8. Verfahren zur Herstellung einer künstlichen Augenlinse (1) nach einem der Ansprüche 1 bis 6, wobei das Verfahren die Schritte umfasst:
- Fertigen des Optikelements (10), wobei das Optikelement (10) das erste Linsenmaterial mit dem ersten Brechungsindex aufweist,
- Einbringen des Optikelements (10) in eine Gussform (70, 72),
- Überspritzen des Optikelements (10) in der Gussform (70, 72) mit dem zweiten Linsenmaterial zur Erzeugung des Berandungselements (50) sowie der scharfen Kante (55), wobei das Berandungselement (50) das zweite Linsenmaterial mit dem zweiten Brechungsindex aufweist, wobei der erste Brechungsindex größer ist als der zweite Brechungsindex.

9. Verfahren zur Herstellung einer künstlichen Augenlinse (1) nach einem der Ansprüche 1 bis 6, wobei das Verfahren die Schritte umfasst:
- Bereitstellen eines Linsenrohlings,
- Herausarbeiten der geometrischen Form des Optikelements (10) der künstlichen Augenlinse (1), wobei das Optikelement (10) das erste Linsenmaterial aufweist,
- Herausarbeiten der geometrischen Form des Berandungselements (50) sowie der scharfen Kante (55) der künstlichen Augenlinse (1), wobei das Berandungselement (50) das zweite Linsenmaterial aufweist,
- Beaufschlagen des Optikelements (10) oder des Berandungselements (50) mit Licht derart, dass der Brechungsindex des ersten oder zweiten Linsenmaterials derart verändert wird, dass der erste Brechungsindex größer ist als der zweite Brechungsindex.

10. Verfahren zur Herstellung einer künstlichen Augenlinse (1) nach einem der Ansprüche 1 bis 6, wobei das Verfahren die Schritte umfasst:
- Bereitstellen eines Linsenrohlings,
- Herausarbeiten der geometrischen Form des Optikelements (10) der künstlichen Augenlinse (1), wobei das Optikelement (10) das erste Linsenmaterial aufweist,
- Herausarbeiten der geometrischen Form und der scharfen Kante (55) des Berandungselements (50), wobei das Berandungselements (50) das zweite Linsenmaterial aufweist,
- Beaufschlagen des Optikelements (10) oder des Berandungselements (50) mit einer Chemikalie derart, dass der Brechungsindex des ersten oder zweiten Linsenmaterials derart verändert wird, dass der erste Brechungsindex größer ist als der zweite Brechungsindex.

## Claims

1. Artificial eye lens (1) for implantation in an eye, comprising:
an optical element (10) having a front side (20), a back side (30), an optical zone (40) and a principal optical axis (A), wherein
- the optical zone (40) is designed to guide light onto a retina of the eye,
and
an edging element (50) that has a rigid connection to the optical element (10) outside the optical zone (40), wherein
- the edging element (50) has a sharp edge (55) in a section plane comprising the principal optical axis (A), with a sharp edge (55) being present if the angle at the edge (55) measured in the section plane within the edging element (50) is less than or equal to 100°, preferably less than or equal to 90°,
wherein the optical element (10) does not have a sharp edge in the section plane in the region of the rigid connection,
**characterized**
- **in that** the optical element (10) comprises a first lens material with a first refractive index,
- **in that** the edging element (50) comprises a second lens material with a second refractive index and
- **in that** the first refractive index is greater than the second refractive index.

2. Artificial eye lens (1) according to Claim 1, wherein the second refractive index differs from the refractive index of aqueous humour by less than 0.05, preferably by less than 0.01 and particularly preferably by less than 0.003.

3. Artificial eye lens (1) according to either of the preceding claims, wherein the second refractive index has a gradient.

4. Artificial eye lens (1) according to any of the preceding claims, wherein the edging element (50) substantially completely encloses the front side (20) and/or the back side (30) of the optical element (10).

5. Artificial eye lens (1) according to Claim 4, wherein the edging element (50) has a sharp edge (55) in substantially any section plane and/or that the optical element (10) has no sharp edge in the region of the rigid connection in substantially any section plane.

6. Artificial eye lens (1) according to any of the preceding claims, wherein the eye lens (1) further comprises a haptic (60) that is designed to secure the eye lens (1) in the eye in the implanted state, wherein the haptic (60) has a refractive index that differs from the refractive index of aqueous humour by less than 0.01, preferably by less than 0.003 and particularly preferably by less than 0.001.

7. Method for producing an artificial eye lens (1) according to any of Claims 1 to 6, wherein the method comprises the following steps:
- fabricating the optical element (10), wherein the optical element (10) comprises the first lens material with the first refractive index,
- fabricating the edging element (50) and fabricating the sharp edge (55) of the edging element (50), wherein the edging element (50) comprises the second lens material with the second refractive index, wherein the first refractive index is greater than the second refractive index,
- connecting the optical element (10) to the edging element (50).

8. Method for producing an artificial eye lens (1) according to any of Claims 1 to 6, wherein the method comprises the steps of:
- fabricating the optical element (10), wherein the optical element (10) comprises the first lens material with the first refractive index,
- introducing the optical element (10) into a mould (70, 72),
- overmoulding the optical element (10) in the mould (70, 72) with the second lens material in order to produce the edging element (50) and the sharp edge (55), wherein the edging element (50) comprises the second lens material with the second refractive index, wherein the first refractive index is greater than the second refractive index.

9. Method for producing an artificial eye lens (1) according to any of Claims 1 to 6, wherein the method comprises the steps of:
- providing a lens blank,
- forming the geometric shape of the optical element (10) of the artificial eye lens (1), wherein the optical element (10) comprises the first lens material,
- forming the geometric shape of the edging element (50) and the sharp edge (55) of the artificial eye lens (1), wherein the edging element (50) comprises the second lens material,
- applying light to the optical element (10) or the edging element (50) such that the refractive index of the first or second lens material is changed such that the first refractive index is greater than the second refractive index.

10. Method for producing an artificial eye lens (1) according to any of Claims 1 to 6, wherein the method comprises the steps of:
- providing a lens blank,
- forming the geometric shape of the optical element (10) of the artificial eye lens (1), wherein the optical element (10) comprises the first lens material,
- forming the geometric shape and the sharp edge (55) of the edging element (50), wherein the edging element (50) comprises the second lens material,
- applying a chemical to the optical element (10) or the edging element (50) such that the refractive index of the first or second lens material is changed such that the first refractive index is greater than the second refractive index.

## Revendications

1. Cristallin oculaire artificiel (1) destiné à être implanté dans un œil, comprenant :
un élément optique (10) avec une face avant (20), une face arrière (30), une zone optique (40) et un axe optique principal (A),
- la zone optique (40) étant formée pour guider la lumière sur la rétine de l'œil,
et
un élément de bordure (50), qui est relié fixement à l'élément optique (10) à l'extérieur de la zone optique (40),
- l'élément de bordure (50) comportant une arête vive (55) dans un plan de coupe, qui comprend l'axe optique principal (A), une arête vive (55) étant présente, lorsque l'angle sur l'arête (55) mesuré dans le plan de coupe à l'intérieur de l'élément de bordure (50) est inférieur ou égal à 100°, de manière préférée est inférieur ou égal à 90°,
l'élément optique (10) ne comportant pas d'arête vive dans la zone de la liaison fixe dans le plan de coupe,
**caractérisé en ce**
- **que** l'élément optique (10) comporte un premier matériau de cristallin avec un premier indice de réfraction,
- **que** l'élément de bordure (50) comporte un deuxième matériau de cristallin avec un deuxième indice de réfraction, et
- **que** le premier indice de réfraction est supérieur au deuxième indice de réfraction.

2. Cristallin oculaire artificiel (1) selon la revendication 1, le deuxième indice de réfraction s'écartant de l'indice de réfraction de l'humeur aqueuse de moins de 0,05, de manière préférée de moins de 0,01, de manière particulièrement préférée de moins de 0,003.

3. Cristallin oculaire artificiel (1) selon l'une des revendications précédentes, le deuxième indice de réfraction comportant un gradient.

4. Cristallin oculaire artificiel (1) selon l'une des revendications précédentes, l'élément de bordure (50) entourant sensiblement complètement la face avant (20) et/ou la face arrière (30) de l'élément optique (10).

5. Cristallin oculaire artificiel (1) selon la revendication 4, l'élément de bordure (50) comportant une arête vive (55) pour sensiblement chaque plan de coupe et/ou que l'élément optique (10) ne comporte aucune arête vive dans la zone de la liaison fixe pour sensiblement chaque plan de coupe.

6. Cristallin oculaire artificiel (1) selon l'une des revendications précédentes, le cristallin oculaire (1) comprenant par ailleurs un élément haptique (60) qui est formé pour fixer le cristallin oculaire (1) dans l'œil à l'état implanté, l'élément haptique (60) présentant un indice de réfraction qui s'écarte de l'indice de réfraction de l'humeur aqueuse de moins de 0,01, de manière préférée de moins de 0,003, de manière particulièrement préférée de moins de 0,001.

7. Procédé de fabrication d'un cristallin oculaire artificiel (1) selon l'une des revendications 1 à 6, le procédé comprenant des étapes suivantes :
- production de l'élément optique (10), l'élément optique (10) comportant le premier matériau de cristallin avec le premier indice de réfraction,
- production de l'élément de bordure (50) et production de l'arête vive (55) de l'élément de bordure (50), l'élément de bordure (50) comportant le deuxième matériau de cristallin avec le deuxième indice de réfraction, le premier indice de réfraction étant supérieur au deuxième indice de réfraction,
- liaison de l'élément optique (10) à l'élément de bordure (50).

8. Procédé de fabrication d'un cristallin oculaire artificiel (1) selon l'une des revendications 1 à 6, le procédé comprenant les étapes :
- production de l'élément optique (10), l'élément optique (10) comportant le premier matériau de cristallin avec le premier indice de réfraction,
- introduction de l'élément optique (10) dans un moule de coulée (70, 72),
- surmoulage de l'élément optique (10) dans le moule de coulée (70, 72) avec le deuxième matériau de cristallin pour générer l'élément de bordure (50) ainsi que l'arête vive (55), l'élément de bordure (50) comportant le deuxième matériau de cristallin avec le deuxième indice de réfraction, le premier indice de réfraction étant supérieur au deuxième indice de réfraction.

9. Procédé de fabrication d'un cristallin oculaire artificiel (1) selon l'une des revendications 1 à 6, le procédé comprenant les étapes :
- fourniture d'une ébauche de cristallin,
- élaboration de la forme géométrique de l'élément optique (10) du cristallin oculaire artificiel (1), l'élément optique (10) comprenant le premier matériau de cristallin,
- élaboration de la forme géométrique de l'élément de bordure (50) ainsi que de l'arête vive (55) du cristallin oculaire artificiel (1), l'élément de bordure (50) comportant le deuxième matériau de cristallin,
- soumission de l'élément optique (10) ou de l'élément de bordure (50) à l'action de la lumière de telle manière que l'indice de réfraction du premier ou du deuxième matériau de cristallin est modifié de telle manière que le premier indice de réfraction est supérieur au deuxième indice de réfraction.

10. Procédé de fabrication d'un cristallin oculaire artificiel (1) selon l'une des revendications 1 à 6, le procédé comprenant les étapes :
- fourniture d'une ébauche de cristallin,
- élaboration de la forme géométrique de l'élément optique (10) du cristallin oculaire artificiel (1), l'élément optique (10) comprenant le premier matériau de cristallin,
- élaboration de la forme géométrique et de l'arête vive (55) de l'élément de bordure (50), l'élément de bordure (50) comportant le deuxième matériau de cristallin,
- soumission de l'élément optique (10) ou l'élément de bordure (50) à l'action d'un produit chimique de telle manière que l'indice de réfraction du premier ou du deuxième matériau de cristallin est modifié de telle manière que le premier indice de réfraction est supérieur au deuxième indice de réfraction.
